(19) Europäisches Patentamt
European Patent Office
Office européen des brevets

(11) Veröffentlichungsnummer: **0 220 417**
**B1**

(12) # EUROPÄISCHE PATENTSCHRIFT

(45) Veröffentlichungstag der Patentschrift: **19.07.89**

(51) Int. Cl.$^4$: **C 07 C 99/00**, C 07 C101/28

(21) Anmeldenummer: **86111763.8**

(22) Anmeldetag: **25.08.86**

(54) **Verfahren zur enantioselektiven Herstellung von alpha-Vinyl-alpha-aminocarbonsäuren.**

(30) Priorität: **10.10.85 DE 3536146**

(43) Veröffentlichungstag der Anmeldung: **06.05.87 Patentblatt 87/19**

(45) Bekanntmachung des Hinweises auf die Patenterteilung: **19.07.89 Patentblatt 89/29**

(84) Benannte Vertragsstaaten: **AT BE CH DE FR GB IT LI LU NL SE**

(56) Entgegenhaltungen:
**EP-A- 0 137 351**
**US-A- 4 147 873**

(73) Patentinhaber: **Degussa Aktiengesellschaft**
**Weissfrauenstrasse 9**
**D-6000 Frankfurt am Main 1 (DE)**

(72) Erfinder: **Seebach, Dieter, Prof.Dr.**
**Orellistrasse 3**
**CH-8044 Zürich (CH)**
Erfinder: **Weber, Theodor**
**Domaine des Barges**
**CH-1896 Vouvry (CH)**

**EP 0 220 417 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung ist ein Verfahren zur enantioselektiven Herstellung von α-vinyl-aminocarbonsäuren der allgemeinen Formel

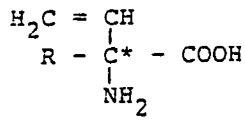

(I)

in der * ein Asymmetriezentrum und R Wasserstoff, eine Methyl-, Ethyl-, n-Propyl-, Isopropyl-, n-Butyl-, Isobutyl-, sek. Butyl-, Allyl-, Benzyl- oder eine in beliebiger Ringstellung 1- bis 3-fach durch eine Alkyl- oder Alkoxygruppe oder durch Fluor oder Chlor substituierte Benzylgruppe bedeuten, welches dadurch gekennzeichnet ist, daß man ein Imidazolidin-4-on der allgemeinen Formel

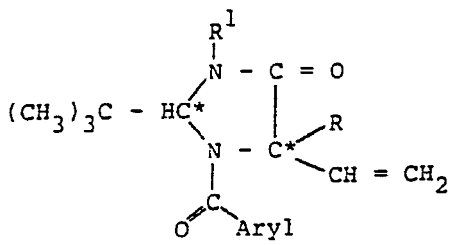

(II)

in der * und R die bereits angegebene Bedeutung haben und $R^1$ eine Methyl- oder Ethylgruppe und Aryl eine Phenyl- oder substituierte Phenylgruppe bedeuten, sauer hydrolysiert.

Aus der EP-A-0 137 351 ist ein Verfahren zur enantioselektiven Herstellung von α-alkylierten acylischen α-Aminocarbonsäuren bekannt, welches dadurch gekennzeichnet ist, daß man ein Imidazolidin-4-on der allgemeinen Formel

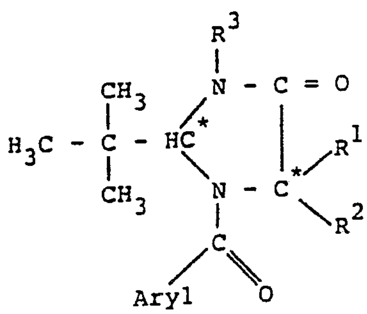

in der * ein Asymmetriezentrum, $R^1$ eine Methyl-, Ethyl-, n-Propyl-, n-Butyl-, Allyl-, Benzyl- oder substituierte Benzylgruppe und $R^2$ eine Methyl-, Ethyl-, n-Propyl-, i-Propyl-, n-Butyl-, sekundäre Butyl-, i-Butyl-, Methoxymethyl-, Methylmercaptomethyl-, 2-Methyl-mercaptoethyl-, 2-Ethylmercaptoethyl-, Phenyl-, Benzyl- oder eine in beliebiger 1- bis 3-fach durch eine Alkyl- oder Alkoxygrupe oder durch Fluor oder Chlor substituierte Benzylgruppe, $R^3$ eine Methyl- oder Ethylgruppe und Aryl eine Phenylgruppe, oder substituierte Phenylgruppe bedeuten, sauer hydrolysiert.

Es ist ferner bereits bekannt, eine 2-Methylmercaptoethylgruppe durch Oxidation und anschließende Fragmentierung in eine Vinylgruppe umzuwandeln. In der US-A-4 147 873 ist beispielsweise im Zusammenhang mit der Herstellung von α-Vinylglutaminsäure die Umwandlung von 5-Carbomethoxy-5-methylthioethyl-2-pyrrolidon in 5-Carboxymethyl-5-vinyl-2-pyrrolidon beschrieben. Das Vinylderivat wird jedoch nur in einer relativ mäßigen Ausbeute erhalten.

Zur Durchführung des erfindungsgemäßen Verfahrens wird zunächst L-Methionin nach dem an sich bekannten, in der EP-A-0 137 351 beschriebenen Verfahren in ein Imidazolidin-4-on der allgemeinen Formel

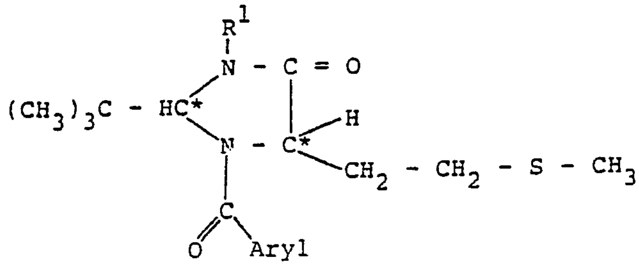

in der *, $R^1$ und Aryl die bereits angegebene Bedeutung haben, umgewandelt und dieses mit einem Oxidationsmittel umgesetzt. Dadurch wird die Thioethergruppe in eine Sulfoxid- bzw. Sulfongruppe überführt. Als Oxidationsmittel können Wasserstoffperoxid in Lösungsmitteln wie Ameisen- oder Essigsäure (« in-situ-Percarbonsäuren ») oder vorgebildete Percarbonsäuren, wie Perameisen-, Peressig-, Perpropionsäure — — letztere z. B. als Lösung in Benzol — oder m-Chlorperbenzoesäure eingesetzt werden. Auch Perjodate können als Oxidationsmittel dienen. Die Oxidationsreaktion erfolgt zweckmäßig bei einer Temperatur zwischen 0 und 50 °C, vorzugsweise zwischen 20 und 30 °C.

Im Falle der Oxidation mit Wasserstoffperoxid kann beispielsweise so verfahren werden, daß man das Imidazolidin-4-on der allgemeinen Formel (VI) in Ameisensäure oder Essigsäure löst, Wasserstoffperoxid in 3- bis 5-fachem molaren Überschuß zudosiert und zur Vervollständigung der Reaktion noch etwa 3 bis 5 Stunden lang nachrührt. Danach wird die Säure, beispielsweise mit Sodalösung, neutralisiert, das Oxidationsprodukt mit einem mit Wasser nicht mischbaren Lösungsmittel, wie Methylenchlorid, extrahiert und das Lösungsmittel abgedampft.

Werden vorgebildete Percarbonsäuren als Oxidationsmittel eingesetzt, wird zweckmäßig so verfahren, daß man das Imidazolidin-4-on der allgemeinen Formel (VI) portionsweise in die jeweilige Percarbonsäure einträgt. Als Verdünnungsmitel können beispielsweise Ameisensäure, Essigsäure oder Benzol dienen. Geeignete Lösungsmittel für m-Chlorperbenzoesäure sind vor allem chlorierte Kohlenwasserstoffe. Anschließend wird wieder die Säure neutralisiert bzw. die in chlorierten Kohlenwasserstoffen unlösliche m-Chlorbenzoesäure abfiltriert, erforderlichenfalls das Oxidationsprodukt extrahiert und das Lösungs- bzw. Extraktionsmittel abgedampft.

Im Falle der Oxidation mit einem Perjodat werden als Lösungs- bzw. Verdünnungsmittel vorzugsweise Mischungen aus Wasser und niederen Alkoholen, wie Methanol, Ethanol oder Propanol, verwendet. Zweckmäßigerweise wird das Imidazolidin-4-on der allgemeinen Formel (VI) in dem Alkohol gelöst und eine wäßrige Lösung des Perjodats, vorzugsweise Natriumperjodat, zugegeben. Nach einer Nachreaktionszeit von etwa 3 bis 5 Stunden die Lösungsmittel abdestilliert, der Rückstand wird in einem mit Wasser nicht mischbaren Lösungsmittel aufgenommen und die Salze werden mit Wasser herausgewaschen. Dann wird die organische Phase eingedampft.

Der jeweils nach dem Abdampfen des Lösungs- bzw. Extraktionsmittels verbleibende Rückstand an rohem Oxidationsprodukt wird dann ohne weitere Reinigung in einem hochsiedenden Lösungsmittel aufgenommen und bei einer Temperatur zwischen 170 und 250 °C, vorzugsweise zwischen 190 und 220 °C, gerührt. Dabei findet die Fragmentierung zu den 5-Vinyl-5H-imidazolidin-4-onen der allgemeinen Formel

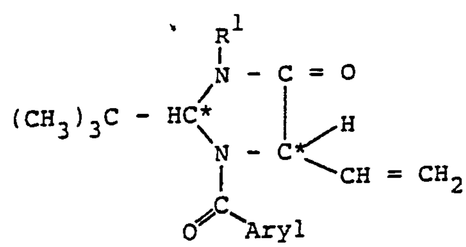

(III)

statt, in der *, $R^1$ und Aryl die bereits angegebene Bedeutung haben. Als hochsiedende Lösungsmittel können beispielsweise gesättigte aliphatische oder alicyclische Kohlenwasserstoffe, Chlorbenzole, Xylole, Mesitylen oder Tetralin dienen. Das Erhitzen kann erforderlichenfalls unter Druck erfolgen. Bei ausreichend hohem Siedepunkt des verwendeten Lösungsmittels kann aber auch unter Normaldruck gearbeitet werden.

Nach beendeter Fragmentierung wird das Lösungsmittel unter vermindertem Druck abgedampft und der verbleibende Rückstand wird durch Flash-Chromatographie gereinigt. Man erhält so die analysenreinen Imidazolidin-4-one der allgemeinen Formel (III) in sehr guten Ausbeuten.

Diese können durch Alkylierung mit einem Alkylierungsmittel der allgemeinen Formel

$$R^2 — X \qquad (IV),$$

in der $R^2$ eine der für R bereits angegebenen Bedeutungen außer Wasserstoff hat und X eine Austrittsgrupe aus der Reihe Chlorid, Bromid, Jodid, Mesylat, Trifluormethylsulfonat oder Tosylat bedeutet, in die Imidazolidin-4-one der allgemeinen Formel (II) umgewandelt werden, in der R eine der bereits angegebenen Bedeutungen außer Wasserstoff hat.

Zur Durchführung der Alkylierung wird das Imidazolidin-4-on der allgemeinen Formel (III) mit einer starken Base der allgemeinen Formel

$$M — Y \qquad (VII),$$

in der M für Lithium, Natrium oder Kalium steht und Y Wasserstoff oder eine n-Butyl-, tert.Butylat-, Amino-, Dimethylamino-, Diethylamino-, Di-n-propylamino-, Di-iso-propylamino-, Di-(trimethylsilyl)-amino- oder

Phenylgruppe bedeutet, umgesetzt. Diese Umsetzung wird zweckmäßigerweise so durchgeführt, daß man die starke Base der allgemeinen Formel (VII) in einer Menge von 1,0 bis 1,1 Äquivalenten auf einmal oder innerhalb weniger Minuten zu einer Lösung des Imidazolidin-4-ons in einem inerten Lösungsmittel zugibt. Geeignete inerte Lösungsmittel sind beispielsweise Ether, wie Diethylether, Di-n-propylether, Methyl-tert.-butylether oder Tetrahydrofuran, und auch Kohlenwasserstoffe, wie n-Pentan, n-Hexan oder Cyclohexan. Gegebenenfalls können auch Gemische aus solchen Ethern und Kohlenwasserstoffen eingesetzt werden.

Als starke Basen der allgemeinen Formel (VII) können beispielsweise Butyllithium, Phenyllithium, Natriumhydrid, Kalium-tert.butylat oder N,N-disubstituierte Lithiumamide Verwendung finden. Bevorzugt werden Butyllithium und Lithiumdiisopropylamid. Gegebenenfalls kann das Lithiumdiisopropylamid in situ aus Butyllithium und N,N-Diisopropylamin hergestellt werden. Die zweckmäßigsten Reaktionstemperaturen liegen zwischen — 80 und 0 °C.

Bei dieser Umsetzung werden intermediär Enolate gebildet, die anschließend direkt, zweckmäßigerweise ebenfalls wieder bei einer Temperatur zwischen — 80 und 0 °C, mit dem Alkylierungsmittel der allgemeinen Formel (IV) weiter umgesetzt werden, das zweckmäßigerweise in einem 1,5- bis 2,5-fachen molaren Überschuß, bezogen auf das ursprünglich eingesetzte Imidazolidin-4-on der allgemeinen Formel (III), angewandt wird.

Bei dieser Alkylierungsreaktion entstehen die Imidazolidin-4-one der allgemeinen Formel (II), in der R eine der bereits angegebenen Bedeutungen außer Wasserstoff hat, in guten chemischen Ausbeuten und stereochemisch praktisch einheitlich.

Alternativ können Imidazolidin-4-one der allgemeinen Formel (II), in der R eine der bereits angegebenen Bedeutungen außer Wasserstoff hat, auch in der Weise hergestellt werden, daß man ein Imidazolidin-4-on der allgemeinen Formel (VI) zunächst, wie oben für die Imidazolidin-4-one der allgemeinen Formel (III) beschrieben, mit einem Alkylierungsmittel der allgemeinen Formel (VI) zu einem Imidazolidin-4-on der allgemeinen Formel

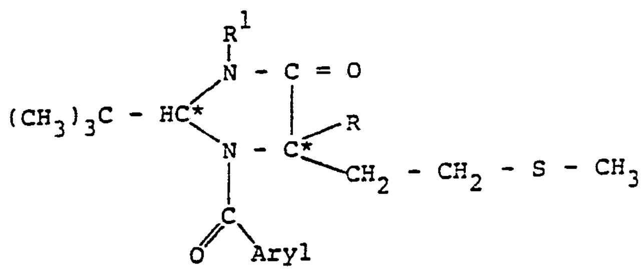

(V)

in der *, $R^1$ und Aryl die bereits angegebene Bedeutung und R eine der bereits angegebenen Bedeutungen außer Wasserstoff haben, umsetzt und dann dieses, wie oben für die Imidazolidin-4-one der allgemeinen Formel (VI) beschrieben, oxidiert und anschließend fragmentiert.

Die nach einer der beschriebenen Verfahrensweisen erhaltenen Imidazolidin-4-one der allgemeinen Formel (II) werden schließlich sauer zu den α-Vinyl-α-aminocarbonsäuren der allgemeinen Formel (I) hydrolysiert. Dies geschieht zweckmäßigerweise durch Erhitzen mit einer relativ konzentrierten Mineralsäure auf eine Temperatur zwischen 150 und 200 °C unter Druck. Als Säuren werden vor allem 10 bis 36 gewichtsprozentige Salzsäure oder 20 bis 48 gewichtsprozentige wäßrige Bromwasserstoffsäure eingesetzt.

Das Hydrolysegemisch wird abgekühlt, erforderlichenfalls filtriert, mit Methylenchlorid extrahiert und die wäßrige Phase im Vakuum eingedampft. Der Rückstand wird in Wasser aufgenommen und in an sich bekannter Weise mit Hilfe eines Ionenaustauschers dehydrohalogeniert. Die verbleibende wäßrige Lösung der α-Vinyl-α-aminocarbonsäure der allgemeinen Formel (I) wird eingedampft und erforderlichenfalls chromatographisch weiter gereinigt. Man erhält so die optisch reinen kristallinen α-Vinyl-α-aminocarbonsäuren.

Das erfindungsgemäße Verfahren eröffnet somit einen hervorragenden Zugang zu optisch reinen α-Vinyl-α-aminocarbonsäuren auf der Basis von L-Methionin. Solche α-Vinyl-α-aminocarbonsäuren gewinnen immer größere Bedeutung wegen ihrer Eigenschaften als Enzyminhibitoren oder als antibiotika.

Das erfindungsgemäße Verfahren wird durch die nachfolgenden Beispiele näher erläutert :

## Beispiel 1

a) Herstellung von (S)-N-(2',2'-Dimethylpropyliden)-methioninmonomethylamid

Zu 40,0 g (247 mmol) (S)-Methioninmonomethylamid, gelöst in 100 ml n-Pentan, wurden 27,5 ml (250 mmol) Pivalaldehyd zugegeben. Das Reaktionsgemisch wurde am Wasserabscheider gekocht, bis

die Wasserbildung beendet war (3 Stunden). Das Lösungsmittel wurde unter vermindertem Druck entfernt und es hinterblieben 52,2 g (92 % der Theorie) an (S)-N-(2',2'-Dimethylpropyliden)-methioninmonomethylamid, das ohne weitere Reinigung weiterverarbeitet wurde.

b) Herstellung von (2S,5S)-1-Benzoyl-2-tert.butyl-3-methyl-5-(3'-thiabutyl)-imidazolidin-4-on

Eine Lösung von 23,0 g (100,0 mmol) des nach a) hergestellten (S)-N-(2',2'-Dimethylpropyliden)-methioninmonomethylamids in 30 ml Methanol wurde unter Kühlung auf 0 °C mit 60 ml einer gesättigten methanolischen Salzsäure versetzt und 30 Minuten bei 0 °C und anschließend 2 Stunden bei 25 °C gerührt. Das Lösungsmittel wurde bei 25 °C unter vermindertem Druck entfernt und der Rückstand in 100 ml Methylenchlorid aufgenommen. Die Methylenchloridlösung wurde bei 0 °C mit 11,6 ml (100 mmol) Benzoylchlorid und 27,7 ml (200 mmol) Triethylamin versetzt. Nach dem Erwärmen auf 25 °C wurde das Reaktionsgemisch zweimal mit je 150 ml 2 N Sodalösung und einmal mit 100 ml Wasser ausgewaschen. Die organische Phase wurde über $MgSo_4$ getrocknet, das Methylenchlorid im Vakuum abdestilliert und der Rückstand 1 Stunde bei 50 °C und 0,065 mbar getrocknet. Ausbeute an (2S,5S)-1-Benzoyl-2-tert.butyl-3-methyl-5-(3'-thiabutyl)-imidazolidin-4-on : 31,5 g (94 % der Theorie). Zur weiteren Reinigung wurde zweimal aus Diethylether umkristallisiert.

Schmelzpunkt : 129 °C

c) Herstellung von (2S,5S)-1-1Benzoyl-2-tert.butyl-3-methyl-5-vinyl-imidazolidin-4-on

Zu einer Lösung von 5,02 g (15 mmol) des nach b) hergestellten (2S,5S)-1-Benzoyl-2-tert.butyl-3-methyl-5-(3'-thiabutyl)-imidazolidin-4-ons in 30 ml Eisessig wurden bei 25 °C 5,7 g (60 mmol) einer 35 gewichtsprozentigen wäßrigen Lösung von Wasserstoffperoxid gegeben und anschließend wurde weitere 4 Stunden lang bei 25 °C gerührt. Das Reaktionsgemisch wurde mit insgesamt 300 ml Methylenchlorid extrahiert. Der Extrakt wurde zweimal mit je 150 ml gesättigter Sodalösung und einmal mit 150 ml Wasser gewaschen. Nach Trocknen der organischen Phase über $MgSO_4$ und Abdampfen des Lösungsmittels wurde der feste Rückstand ohne weitere Reinigung mit ca. 60 ml Xylol versetzt und in einem Einschlußrohr 2 Stunden auf 200 bis 210 °C erhitzt.

Das Xylol wurde unter vermindertem Druck abdestilliert und der Rückstand wurde durch Flash-Chromatographie (Ether/$CH_2Cl_2$ im Volumenverhältnis 8 : 1) gereinigt. Es ergaben sich 3,20 g (78 % der Theorie) and (2S,5S)-1-Benzoyl-2-tert.butyl-3-methyl-5-vinyl-imidazolidin-4-on, das zur weiteren Reinigung aus einem Gemisch $CH_2Cl_2$/Pentan im Volumenverhältnis 1 : 1 umkristallisiert wurde.

Schmelzpunkt : 169 bis 171 °C

$[\alpha]^{25}_D$ : + 110,0° (c = 1 ; $CHCl_3$)

IR (KBr) : 2 990s, 1 710s, 1 650s, 1 380s $cm^{-1}$

$^1$H-NMR ($CDCl_3$) : 7,65-7,30 (m, 5H aromatisch) ; 5,69 (s, 1H, H—C(2)) ; 5,30-4,55 (m, 4H, vinyl) ; 3,12 (s, 1H, $H_3C$—N) ; 1,10 ppm (S, 9H, t-Butyl).

d) Herstellung von (2S,5R)-1-Benzoyl-2-tert.butyl-3,5-dimethyl-5-vinyl-imidazolidin-4-on

Zu einer —78 °C kalten Lösung von 6,5 mmol Diisopropylamin in 20 ml Tetrahydrofuran wurden 5,5 mmol n-Butyllithium langsam zugegeben. Nach 30 Minuten wurde die so erhaltene Lithiumdiisopropylamid-Lösung bei —78 °C zu einer Lösung von 1,42 g (5 mmol) des nach c) hergestellten (2S,5S)-1-Benzoyl-2-tert.butyl-3-methyl-5-vinyl-imidazolidin-4-ons in 20 ml Tetrahydrofuran zugetropft. Dabei färbte sich die anfangs farblose Lösung tief rot. Nach 30 Minuten Rühren bei —78 °C wurden 0,78 ml (12,5 mmol) Methyljodid zugegeben und es wurde weitere 5 Stunden bei 0 °C gerührt. Das leicht gelbe Reaktionsgemisch wurde in 200 ml gesättigte wäßrige Ammoniumchlorid-Lösung gegossen. Dann wurde mit insgesamt 250 ml Methylenchlorid extrahiert und die organische Phase wurde mit 150 ml Kochsalz-Lösung gewaschen.

Nach Trocknen der organischen Phase über Magnesiumsulfat wurde das Lösungsmittel bei 40 mbar am Rotationsverdampfer abgezogen und der feste Rückstand wurde aus $CH_2Cl_2$/Pentan im Volumenverhältnis 1 : 1 umkristallisiert. Es ergaben sich 1,24 g (83 % der Theorie) an (2S,5R)-1-Benzoyl-2-tert.butyl-3,5-dimethyl-5-vinyl-imidazolidin-4-on.

Schmelzpunkt : 117 bis 117,5 °C

$[\alpha]^{25}_D$ : — 58,3° (c = 1 ; $CHCl_3$)

IR (KBr) : 2 980 m, 2 880 m, 1 700 s, 1 640 s, 1 605 m, 1 585 m $cm^{-1}$

$^1$H-NMR ($CDCl_3$) : 7,58-7,30 (m, 5H aromatisch) ; 6,21 (dd, $J_1$ = 11, $J_2$ = 18, 1H, CH—C(5)) ; 5,79 (s, 1H, H—C(2)) ; 5,20 (dd, $J_1$ = 11, $J_2$ =18 , 2H, $H_2C$ = c(5)) ; 3,08 (s, 3H, $H_3C$—N) ; 1,16 (s, 3H, $H_3C$—C(5)) ; 1,10 ppm (s, 9H, t. Butyl).

e) Herstellung von (R)-2-Vinyl-alanin

Eine Mischung aus 0,493 g (1,64 mmol) des nach d) hergestellten (2S,5R)-1-Benzoyl-2-tert.butyl-3,5-dimethyl-5-vinyl-imidazolidin-4-ons und ca. 15 ml 6N Salzsäure wurde im Bombenrohr 4 Stunden auf

160 °C erhitzt. Die wäßrige Phase wurde nach Extraktion mit 30 ml $CH_2Cl_2$ am Rotationsverdampfer eingedampft. Das rohe Hydrochlorid wurde in wenig Wasser aufgenommen und mittels eines Ionenaustauschers (Dowex 50W × 8) dehydrohalogeniert. Das reine (R)-2-Vinyl-alanin wurde nach einer Flash-Chromatographie ($H_3OH/CH_2Cl_2/NH_3$ im Volumverhältnis 5 :5 :0,5) und Eindampfen des Eluates als weiße kristalline Verbindung in einer Ausbeute von 37 % der Theorie erhalten.

Schmelzpunkt : 228 bis 229 °C

$[\alpha]_D^{25}$ : — 32,8 (c = 0,733 ; $H_2O$)

$^1$H-NMR ($D_2O$) (HDO = 4,7 ppm) : 6,20 — 5,80 (m, 1H, <u>CH</u> = $CH_2$) ; 5,60 — 5,20 (m, 2H, CH—$CH_2$) ; 1,52 ppm (s, 3H, $CH_3$).

## Beispiel 2

a) Herstellung von (S)-N-(2′,2′-Dimethylpropyliden)-methioninmonomethylamid

Wie im Beispiel 1a)

b) Herstellung von (2S,5S)-1-Benzoyl-2-tert.butyl-3-methyl-5-(3′-thiabutyl)-imidazolidin-4-on

Wie im Beispiel 1b)

c) Herstellung von (2S,5R)-1-Benzoyl-2-tert.butyl-3,5-dimethyl-5-(3′-thiabutyl)-imidazolidin-4-on

Zu 3,34 g (10,0 mmol) des nach b) hergestellten (2S,5S)-1-Benzoyl-2-tert.butyl-3-methyl-5-β′-thiabutyl)-imidazolidin-4-ons, gelöst in 60 ml Tetrahydrofuran, wurden bei — 60 °C 10,6 mmol einer 1M Lösung von Lithiumdiisopropylamid in Tetrahydrofuran zugegeben. Dabei färbte sich die Lösung tiefrot. Nach weiterem 15-minütigen Rühren bei — 60 °C wurden 0,9 ml (15,0 mmol) Methyljodid zugegeben. Das jetzt schwach gelbe Reaktionsgemisch wurde auf Raumtemperatur erwärmen gelassen, in 100 ml etwa halbgesättigter wäßriger $NH_4Cl$-Lösung gegossen und mit insgesamt 200 ml Diethylether extrahiert. Die vereinigten organischen Extrakte wurde mit Wasser gewaschen über $MgSO_4$ getrocknet und unter vermindertem Druck vom Lösungsmittel befreit. Der Rückstand wurde aus einem Gemisch von Diethylether und n-Pentan im Volumenverhältnis 1 :1 umkristallisiert, wonach 2,36 g (66 % der Theorie) an (2S,5R)-1-Benzoyl-2-tert.butyl-3,5-dimethyl-5-(3′-thiabutyl)-imidazolidin-4-on erhalten wurden.

Schmelzpunkt : 105 °C

$[\alpha]_D^{20}$ : — 71,9° (c =1 ; $CHCl_3$)

d) Herstellung von (2S,5R)-1-Benzoyl-2-tert.butyl-3,5-dimethyl-5-vinyl-imidazolidin-4-on

Zu einer Lösung von 1,045 g (3 mmol) des nach c) hergestellten (2S,5R)-1-Benzoyl-2-tert.butyl-3,5-dimethyl-5-(3′-thiabutyl)-imidazolidin-4-ons in 10 ml Methanol würden 0,666 g (3,32 mmol) Natriumperjodat, gelöst in 4,5 ml Wasser, zudosiert und anschließend wurde 4 Stunde bei 25 °C gerührt. Nach Abdestillieren der Lösungsmittel am Rotationsverdampfer wurde der Rückstand mit 30 ml $CH_2Cl_2$ versetzt, mit 10 ml Wasser ausgerührt und die organische Phase wurde erneut eingedampft. Der feste Rückstand wurde direkt in 10 ml Xylol aufgenommen und 2 Stunden im Bombenrohr auf 210 °C erhitzt. Nach Abdampfen des Lösungsmittels und Flash-Chromatographie (Ether/Petrolether im Volumenverhältnis 2 : 1) wurden 0,852 g (95 % der Theorie) an diastereomerenreinem (2S,5R)-1-Benzoyl-2-tert.butyl-3,5-dimethyl-5-vinyl-imidazolidin-4-on isoliert.

$C_{18}H_{24}N_2O_2$ (300,40)

| | | | | |
|---|---|---|---|---|
| Berechnet : | C 71,96 | H 8,05 | N 9,32 | % |
| Gefunden : | C 71,73 | H 7,94 | N 9,13 | % |

e) Herstellung von (R)-2-Vinyl-alanin

Wie im Beispiel 1e)

## Beispiel 3

a) Herstellung von (S)-N-(2′,2′-Dimethylpropyliden)-methioninmonomethylamid

Wie im Beispiel 1a)

b) Herstellung von (2S,5S)-1-Benzoyl-2-tert.butyl-3-methyl-5-(3′-thiabutyl)-imidazolidin-4-on

Wie im Beispiel 1b)

c) Herstellung von (2S,5S)-1-Benzoyl-2-tert.butyl-3-methyl-5-vinyl-imidazolidin-4-on

Wie im Beispiel 1c)

d) Herstellung von (2S,5R)-1-Benzoyl-5-benzyl-2-tert.butyl-3-methyl-5-vinyl-imidazolidin-4-on

Aus 1,43 g (5 mmol) des nach c) hergestellten (2S,5S)-1-Benzoyl-2-tert.butyl-3-methyl-5-vinyl-imidazolidin-4-ons und 1,49 ml (12,5 nmol) Benzylbromid wurden analog zu Beispiel 1d) nach 4 stündiger Reaktionszeit bei — 5 °C 1,18 g (75 % der Theorie) an (2S,5R)-1-Benzoyl-5-benzyl-2-tert.butyl-3-methyl-5-vinyl-imidazolidin-4-on in einer Diastereomerenreinheit von 87 % gewonnen.
Schmelzpunkt : 152 °C
$[\alpha]_D^{25}$ : — 27,5 °C (c = 1 ; $CHCl_3$)
$C_{24}H_{28}N_2O_2$ (376,50)

| | | | | |
|---|---|---|---|---|
| Berechnet : | C 76,56 | H 7,50 | N 7,44 | % |
| Gefunden ; | C 76,50 | H 7,53 | N 7,46 | % |

MS (EI) : 361 ($M^+$—15, 0,3)
105 (100)
IR (KBr) : 2 870 m, 1 700 s, 1 630 s, 1 595 w, 1 570 m $cm^{-1}$.

e) Herstellung von (R)-2-Vinyl-phenylalanin

Aus einer Mischung von 0,69 g (1,8 mmol) des nach d) hergestellten (2S,5R)-1-Benzoyl-5-benzyl-2-tert.butyl-3-methyl-5-vinyl-imidazolidin-4-ons und 20 ml 6N Salzsäure wurden nach 6-stündigem Erhitzen im Bombenrohr bei 180 °C nach Aufarbeitung analog zu Beispiel 1e) und Flash-Chromatographie ($CH_3OH/CH_2CH_2/NH_3$ im Verhältnis 6 :4 :0,5) 20 mg (5 % der Theorie) an (R)-2-Vinyl-phenylalanin erhalten.
$^1$H-NMR ($D_2O$) : 7,31 (m, 5H aromatisch) ; 6,13 (dd, $J_1$ = 11, $J_2$ = 18. 1H, H-C(3)) ; 5,29 (m, 2H, $H_2C(4)$) ; 3,21 ppm (AB, J = 15, 2H-Benzyl).

## Patentansprüche

1. Verfahren zur enantioselektiven Herstellung von α-vinyl-α-aminocarbonsäuren der allgemeinen Formel

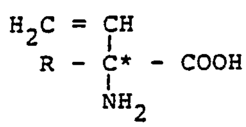

(I)

in der * ein Asymmetriezentrum und R Wasserstoff, eine Methyl-, Ethyl-, n-Propyl-, Isopropyl-, n-Butyl-, Isobutyl-, sek.Butyl-, Allyl-, Benzyl- oder eine in beliebiger Ringstellung 1- bis 3-fach durch eine Alkyl- oder Alkoxygruppe oder durch Fluor oder Chlor substituierte Benzylgruppe bedeuten, dadurch gekennzeichnet, daß man ein Imidazolidin-4-on der allgemeinen Formel

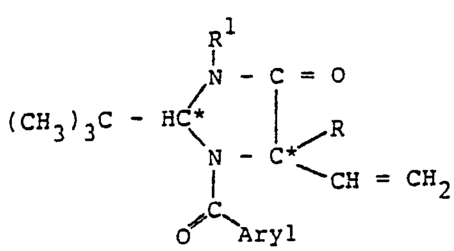

(II)

in der * und R die bereits angegebene Bedeutung haben und $R^1$ eine Methyl- oder Ethylgruppe und Aryl eine Phenyl- oder substituierte Phenylgruppe bedeuten, sauer hydrolysiert.

2. Verfahren nach Anspruch 1, dadurch gekennzeichnet, daß man ein Imidazolidin-4-on der allgemeinen Formel (II), in der R eine der bereits angegebenen Bedeutungen außer Wasserstoff hat, einsetzt, das nach Zusatz einer starken Base durch Umsetzung eines 5-vinyl-5H-imidazolidin-4-ons der allgemeinen Formel

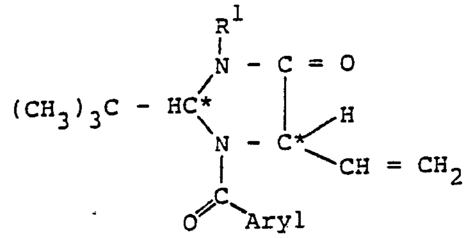

(III)

in der *, $R^1$ und Aryl die bereits angegebene Bedeutung haben, mit einem Alkylierungsmittel der allgemeinen Formel

$$R^2—X \qquad (IV),$$

in der $R^2$ eine der für R bereits angegebenen Bedeutungen außer Wasserstoff hat und X eine Austrittsgruppe aus der Reihe Chlorid, Bromid, Jodid, Mesylat, Trifluormethylsulfonat oder Tosylat bedeutet, hergestellt worden ist.

3. Verfahren nach Anspruch 1, dadurch gekennzeichnet, daß man ein Imidazolidin-4-on der allgemeinen Formel (II) einsetzt, das durch Oxidation und anschließende Fragmentierung eines Imidazolidin-4-ons der allgemeinen Formel

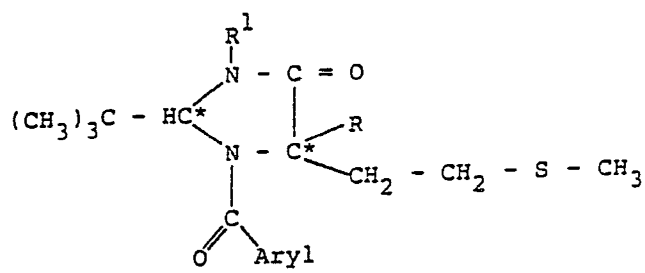

(V)

in der *, R, $R^1$ und Aryl die bereits angegebene Bedeutung haben, hergestellt worden ist.

4. Verfahren nach Anspruch 2, dadurch gekennzeichnet, daß man ein Imidazolidin-4-on der allgemeinen Formel (III) einsetzt, das durch Oxidation und anschließende Fragmentierung eines Imidazolidin-4-ons der allgemeinen Formel

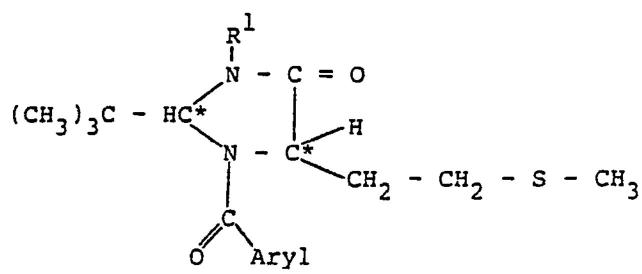

(VI)

in der *, $R^1$ und Aryl die bereits angegebene Bedeutung haben, hergestellt worden ist.

## Claims

1. A process for the enantioselective production of α-vinyl-α-aminocarboxylic acids corresponding to the following general formula

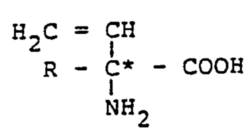

(I)

in which * is an asymmetry centre and R is hydrogen, a methyl, ethyl, n-propyl, isopropyl, n-butyl, isobutyl, sec.-butyl, allyl or benzyl group or a benzyl group substituted 1 to 3 times in any position of the ring by an alkyl or alkoxy group or by fluorine or chlorine, characterized in that an imidazolidin-4-one corresponding to the following general formula

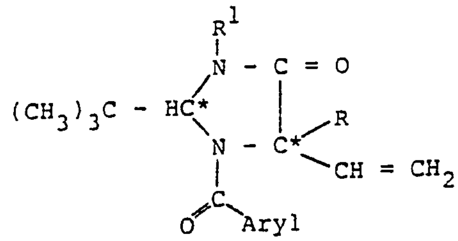

(II)

in which * and R are as already defined and $R^1$ is a methyl or ethyl group and Aryl is a phenyl or substituted phenyl group, is subjected to acidic hydrolysis.

2. A process as claimed in claim 1, characterized in that an imidazolidin-4-one corresponding to general formula (II), in which R has the meanings already defined except for hydrogen, is used which, after the addition of a strong base, has been prepared by reaction of a 5-vinyl-5H-imidazolidin-4-one corresponding to the following general formula

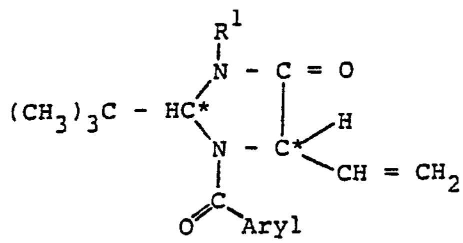

(III)

in which *, $R^1$ and Aryl are as already defined, with an alkylating agent corresponding to the general formula

$$R^2—X \quad (IV)$$

in which $R^2$ has one of the meanings already defined for R except for hydrogen and X is a leaving group from the group consisting of chloride, bromide, iodide, mesylate, trifluoromethyl sulfonate or tosylate.

3. A process as claimed in claim 1, characterized in that an imidazolidin-4-one corresponding to general formula (II) is used which has been prepared by oxidation and subsequent fragmentation of an imidazolidin-4-one corresponding to the following general formula

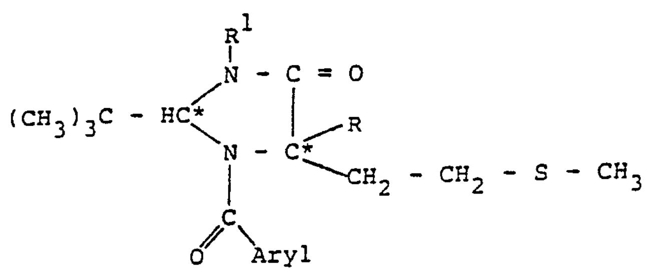

(V)

in which *, R, $R^1$ and Aryl are as already defined.

4. A process as claimed in claim 2, characterized in that an imidazolidin-4-one corresponding to general formula (III) is used which has been obtained by oxidation and subsequent fragmentation of an imidazolidin-4-one corresponding to the following general formula

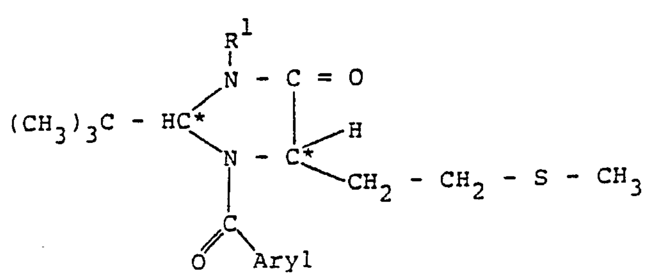

(VI)

in which *, $R^1$ and Aryl are as already defined.

**Revendications**

1. Procédé pour la préparation sélective d'énantiomères d'acides α-vinyl-α-aminocarboxyliques de formule générale

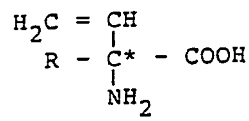

(I)

dans laquelle l'astérisque (*) désigne un centre d'asymétrie et R représente un atome d'hydrogène, un groupe méthyle, éthyle, n-propyle, isopropyle, n-butyle, isobutyle, sec-butyle, allyle, benzyle ou un groupe benzyle substitué de 1 à 3 fois, en position quelconque sur le noyau, par un ou des radicaux alkyle ou alcoxy ou par un ou des atomes de fluor ou de chlore, procédé caractérisé en ce que l'on hydrolyse par voie acide une imidazolidine-4-one de formule générale

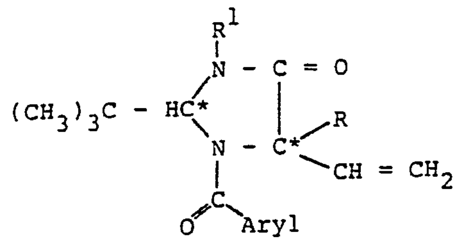

(II)

dans laquelle l'astérisque (*) et R ont les significations déjà données et $R^1$ représente un groupe méthyle ou éthyle et « aryle » représente un groupe phényle ou phényle substitué.

2. Procédé selon la revendication 1, caractérisé en ce que l'on utilise une imidazolidine-4-one de formule générale (II), dans laquelle R a la signification déjà donnée, à l'exception de celle d'un atome d'hydrogène, qui a été préparée, après addition d'une base forte, par réaction d'une 5-vinyl-5H-imidazolidine-4-one de formule générale

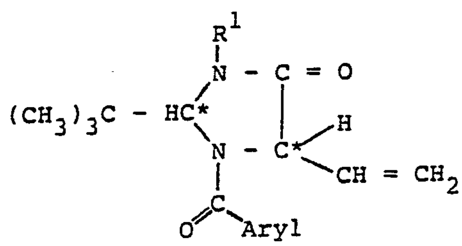

(III)

dans laquelle l'astérique (*), $R^1$ et « aryle » ont les significations déjà données, avec un agent d'alkylation de formule générale

$$R^2—X \qquad (IV)$$

dans laquelle $R^2$ a une des significations déjà données pour R à l'exception de celle d'un atome d'hydrogène, et X représente un groupe séparable choisi parmi un chlorure, bromure, iodure, mésylate, trifluorométhylsulfonate ou tosylate.

3. Procédé selon la revendication 1, caractérisé en ce que l'on utilise une imidazolidine-4-one de formule générale (II) qui a été préparée par oxydation et fragmentation subséquente d'une imidazolidine-4-one de formule générale :

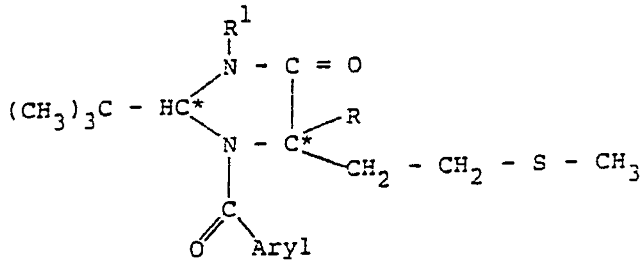

(V)

dans laquelle l'astérisque (*), R, $R^1$ et « aryle » ont les significations déjà données.

4. Procédé selon la revendication 2, caractérisé en ce que l'on utilise une imidazolidine-4-one de formule générale (III), qui a été préparée par oxydation et fragmentation subséquente d'une imidazolidine-4-one de formule générale

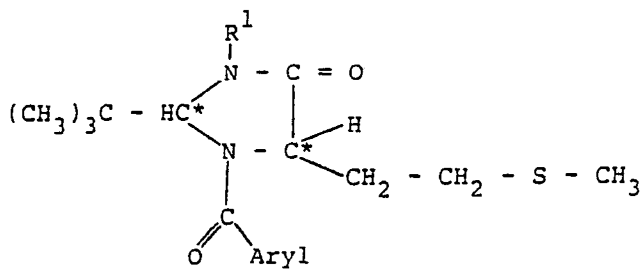

(VI)

dans laquelle l'astérique (*), $R_1$ et « aryle » ont les significations déjà données.